# EUROPEAN PATENT SPECIFICATION

(11) **EP 0 808 828 B1**
(45) Date of publication and mention of the grant of the patent: **12.12.2001**
(21) Application number: 97108220.1
(22) Date of filing: 21.05.1997
(51) Int. Cl.: C07C 267/00, C08K 5/29

(54) **Carbodiimide having double bonds**
Carbodiimide mit Doppelbindungen
Carbodiimides contenant des liaisons doubles

(30) Priority: 21.05.1996 JP 14989896
(43) Date of publication of application: 26.11.1997
(73) Proprietor: NISSHINBO INDUSTRIES, INC., Chuo-ku, Tokyo 103 (JP)
(72) Inventor: Imashiro, Yasuo, 1-18-1 Nishiaraisakaecho Adachi-ku Tokyo (JP); Yamane, Takeshi, 1-18-1 Nishiaraisakaecho Adachi-ku Tokyo (JP); Takahashi, Ikuo, 1-18-1 Nishiaraisakaecho Adachi-ku Tokyo (JP); Horie, Naofumi, 1-18-1 Nishiaraisakaecho Adachi-ku Tokyo (JP)
(74) Representative: Laufhütte, Dieter, Dr.-Ing.

(56) References cited:
- WO-A-93/22282
- DE-A- 2 711 956
- US-A- 5 105 010
- US-A- 5 371 148

## Description

The present invention relates to a novel carbodiimide having olefinic double bonds, and more particularly, the present invention relates to a novel carbodiimide which exhibits excellent performance as a hydrolysis stabilizer for an ester bond-containing resin or as a cross-linking agent for a resin having a group capable of reacting with carbodiimide group or for a compound having an olefinic double bond.

### Description of Related Art

Carbodiimides having a structure represented by the formula

-N=C=N-

have been widely used as a hydrolysis stabilizer for ester group-containing compounds or as a cross-linking agent for resins having a carboxyl group which is a group capable of reacting with carbodiimide group, for example, a cross-linking agent for acrylic resins, based on the high reactivity of the carbodiimide group.

However, carbodiimides which have heretofore been employed in the above-mentioned applications are merely added to resins, and hence, are disadvantageous in that they bleed out of the resins and the desired performance is not obtained in some cases. In addition, the carbodiimide that bled out contaminates a material or the like adjacent to the resin. Moreover, when the amount of the carbodiimide added is increased for obtaining the desired performance, there is such a problem that the carbodiimide comes to function as a plasticizer and the original performance of the resin is thereby deteriorated.

In particular, when the carbodiimide is used as a cross-linking agent for a hydrophilic resin having hydrophilic groups such as an acrylic resin and urethane resin, the carbodiimide must be hydrophilic as well, and hence, when the cross-linking effect is low, the carbodiimide dissolves together with the hydrophilic resin in water, whereby the water resistance and washing resistance of the resin are deteriorated.

Furthermore, in order to enhance the cross-linking effect, an effort has been made to branch the carbodiimide resin or reduce the carbodiimide equivalent (the weight [g] of a carbodiimide compound necessary for giving one mole of carbodiimide group). However, when the branching is too much, the steric hindrance of the carbodiimide becomes too great and the reactivity of the carbodiimide is lowered, so that there is a limit in the branching. Moreover, the inexpensive starting material (isocyanate) for obtaining the carbodiimide is limited in kind, and hence, there is a limit to the effort to reduce the carbodiimide equivalent.

The above-mentioned prior art is disclosed in JP 06192206 A and JP 63264128 A.

WO 9322282 A discloses polymers containing units derived from the alphamethylstyryl group containing carbodiimide to be useful as polymeric dehydrating agents or can be used to cross-link other polymers. Further, polycarbodiimides are described to be useful in coatings.

### SUMMARY OF THE INVENTION

The main object of the present invention is to overcome the above-mentioned difficulties of prior art and provide a novel carbodiimide having at least two olefinic double bonds which is polymerized with, when added to, an ester group-containing resin, for example, a urethane resin, polyethylene terephthalate (PET), polybutylene terephthalate (PBT) or an unsaturated polyester; or a resin having a group capable of reacting with carbodiimide, for example, an acrylic resin, or natural rubber or synthetic rubber supplied in a form of emulsion (latex), to cross-link the resin reticularly without deteriorating the performance of the resin or without bleeding out of the resin, and hence, can be advantageously employed as a hydrolysis stabilizer or cross-linking agent for the resin.

Other objects and advantages of the present invention will become apparent from the following description.

According to the present invention, there is provided a carbodiimide having double bonds represented by the formula (1):

R₁-X-(R₂-NCN)ₙ-R₂-X-R₃ (1)

wherein R₁ and R₃ may be the same as or different from each other and each represents the organic residue having 3 to 40 carbon atoms, of a compound having at least one olefinic double bond and a functional group capable of reacting with an isocyanate group, formed by removing the functional group therefrom; R₂ represents the organic residue of a diisocyanate formed by removing the isocyanate group therefrom; plural R₂ groups may be different from one another; X represents a bond formed by reaction between the above isocyanate group and the above functional group capable of reacting with isocyanate group; and n represents an integer of 1 to 20.

According to the present invention, there is further provided a hydrolysis stabilizer for an ester bond-containing resin, which comprises as a main component a carbodiimide having double bonds represented by the above formula (1).

According to the present invention, there is still further provided a cross-linking agent for a resin having a group capable of reacting with carbodiimide group or for a compound having an olefinic double bond(s), which comprises as a main component a carbodiimide having double bonds represented by the above formula (1).

### DETAILED DESCRIPTION OF THE INVENTION

The carbodiimide having double bonds of the present invention is represented by the above formula (1), and in the formula (1), R₁ and R₃ each represents the organic residue having 3 to 40 carbon atoms of a compound having at least one olefinic double bond and a functional group capable of reacting with isocyanate group formed by removing the functional group from the compound. Incidentally, R₁ and R₃ may be the same as or different from each other

As the compound having at least one olefinic double bond and a functional group capable of reacting with isocyanate group from which R₁ and R₃, which are the above organic residues, are derived (said compound is referred to hereinafter as "double bond compound" in some cases), there can be mentioned specifically 2-hydroxyethyl acrylate, 2-hydroxyethyl methacrylate, 2-hydroxypropyl acrylate, 2-hydroxypropyl methacrylate, pentaerythritol triacrylate, pentaerythritol trimethacrylate, glycidyl acrylate, glycidyl methacrylate, allyl alcohol, 3-buten-1-ol, 4-penten-1-ol, allylamine, N-methylallylamine, N-ethyl-2-methylallylamine, diallylamine, allylcyclohexylamine, butadiene monoxide, 1,2-epoxy-5-hexene, 1,2-epoxy-7-octene, allyl glycidyl ether, 2-allylphenol, 2-allyloxyethanol, pentaerythritol triallyl ether, polyethylene glycol monomethacrylate, polypropylene glycol monomethacrylate, polyethylene glycol monoacrylate, 2-sulfoethyl methacrylate, 2-hydroxy-1,3-dimethacryloxypropane, polypropylene glycol monoacrylate and the like. Accordingly, R₁ and R₃ are the organic residues formed by removing the functional group capable of reacting with isocyanate group from these double bond compounds.

In the above formula (1), R₂ represents the organic residue of a diisocyanate formed by removing the isocyanate group therefrom, and specifically represents the organic residue formed by removing the isocyanate group from an aliphatic diisocyanate such as 4,4'-dicyclohexylmethane diisocyanate, tetramethylxylylene diisocyanate, isophorone diisocyanate or the like, or from an aromatic diisocyanate such as 4,4'-diphenylmethane diisocyanate, tolylene diisocyanate, triisopropylidenephenyl diisocyanate or the like. Incidentally, R₂ may be the residue resulting from one kind of diisocyanate or from a mixture of different kinds of diisocyanates, for example, a mixture of 4,4 '-dicyclohexylmethane diisocyanate, tetramethylxylylene diisocyanate and isophorone diisocyanate, or the like.

In the above formula (1), X represents a bond formed by reaction between the above-mentioned isocyanate group and the above-mentioned functional group capable of reacting with isocyanate group, and specifically represents a urethane bond, a urea bond, an imide bond or an amide bond, and n represents an integer of 1 to 20.

In the production of the present carbodiimide having double bonds and having such a structure as mentioned above, first of all, a carbodiimide-modified diisocyanate represented by the formula (2):

OCN-(R₂-NCN)ₙ-R₂-NCO (2)

(wherein R₂ and n are as defined above) is produced by heating a corresponding diisocyanate in the presence of a carbodiimidization catalyst.

As the above-mentioned carbodiimidization catalyst, suitable is an organic phosphorus compound, and phosphorene oxides are particularly preferable in respect of activity. Specifically, there can be mentioned 3-methyl-1-phenyl-2-phosphorene-1-oxide, 3-methyl-1-ethyl-2-phosphorene-1-oxide, 1,3-dimethyl-2-phosphorene-1-oxide, 1-phenyl-2-phosphorene-1-oxide, 1-ethyl-2-phosphorene-1-oxide, 1-methyl-2-phosphorene-1-oxide and double bond isomers thereof. Among them, 3-methyl-1-phenyl-2-phosphorene-1-oxide is particularly preferable because it is easy to obtain commercially.

The above-mentioned carbodiimidization reaction can be carried out in a known manner. For example, it is sufficient to heat and stir any aliphatic diisocyanate at a reaction temperature within the range of from 150°C to 200°C in a solvent inert thereto or in the absence of any solvent, in a stream of an inert gas such as nitrogen or the like or while bubbling the inert gas, in the presence of the above-mentioned catalyst added in a proportion of 0.1 to 10% by weight (a larger amount is possible when the economy is neglected), preferably 0.5 to 5% by weight, based on the total weight of the isocyanates, to allow carbodiimidization accompanied by decarboxylation to proceed.

When an aromatic diisocyanate is used, it is sufficient to heat the same and stir the aromatic diisocyanate at a reaction temperature within the range of from 20°C to 80°C in the presence of the above catalyst added in an amount of 0.01 to 1% by weight, preferably 0.01 to 0.5% by weight, in the same manner as above.

In the above carbodiimidization reaction, the degree of polymerization of the carbodiimide obtained can be controlled by tracing the degree of polymerization of the carbodiimide under reaction at any time by measuring the amount of the remaining isocyanate by a titration method and cooling the reaction system to, for example, 30°C in the stage that a carbodiimide having the desired degree of polymerization has been produced, to terminate the reaction, thereby obtaining a carbodiimide having the degree of polymerization in said stage. When the desired degree of polymerization is reached, an endblocking agent (an unsaturated organic compound having a functional group reactive with isocyanate) may be introduced into the reaction system.

Subsequently, the carbodiimide-modified diisocyanate obtained is reacted with the above-mentioned double bond compound in an amount equivalent to the diisocyanate by heating the two compounds in solution of a solvent inert thereto or in the absence of any solvent, to obtain the present carbodiimide having double bonds. Incidentally, in this case, a urethanization catalyst or a urethane-urea catalyst may be added to the reaction system.

The solvent which can be used in the reaction between the carbodiimide-modified diisocyanate and the double bond compound may be any solvent as far as it is inert to the isocyanate, the carbodiimide and the functional group of an unsaturated organic compound and can dissolve the carbodiimide and the unsaturated organic compound. Though the solvent is not critical, preferable solvents are, for example, aromatic hydrocarbons such as toluene, benzene, xylene and the like; halogenated hydrocarbons such as chloroform, dichloroethane, Perclene and the like; and ethers such as THF and the like, and when tetramethylxylylene diisocyanate is used, ketones such as acetone and the like; amides such as DMF and the like; etc. can be used as the solvent.

Incidentally, as the above-mentioned urethanization catalyst and urethane-urea catalyst, there may be used those which are generally used and the catalysts are not critical. For example, amine type catalysts are preferable because they have a high activity.

The completion of the above reaction can be confirmed by the disappearance of isocyanate group which is observed at 2200-2300 cm⁻¹ in an infrared absorption spectrum (IR). The reaction temperature and reaction time can be appropriately varied depending upon, for example, the reactivity of the carbodiimide-modified diisocyanate with the double bond compound, the kind and amount of the catalyst used, and the boiling point of the double bond compound; however, the reaction temperature is preferably 0°C to 180°C, more preferably 20°C to 160°C. When the reaction temperature is too high or the reaction time is too long, the production becomes commercially disadvantageous, and it follows in some cases that the olefinic double bonds polymerize. Therefore, such conditions are not desirable.

Moreover, during the synthesis and storage, it is a matter of course to intercept a light for avoiding the polymerization, and in some cases, an adequate polymerization inhibitor may be added. However, the presence of oxygen inhibits the polymerization, so that the polymerization inhibitor is not always necessary in the presence of oxygen.

That the present carbodiimide having double bonds is represented by the above formula (1) can be confirmed by an analysis of IR, a nuclear magnetic resonance spectrum (NMR), a gel-permeation chromatography (GPC) or the like.

As already explained above, that the isocyanate and the functional group of the unsaturated compound have been reacted quantitatively can be confirmed based on the disappearance of absorption of isocyanate confirmed by the IR analysis.

Moreover, the presence of carbodiimide group can be confirmed by the absorption at 2100-2200 cm⁻¹, and absorption of the bond formed by the reaction between the double bond compound and the isocyanate group of the carbodiimide can be confirmed, and, for example, in the case of the reaction between the epoxy group, alcohol group or amino group of the double bond compound and the isocyanate group, absorption of the bond formed by the reaction can be confirmed at 1700-1800 cm⁻¹, 1700-1800 cm⁻¹ or 1400-1700 cm⁻¹, respectively.

In the NMR analysis of proton, the protons of H₂C=CH- and H₂C=C(R)- are observed at 4.5-7 ppm and, by comparing the integration value thereof with the integration value of other protons of methyl, methylene and the like, there can be confirmed the presence of double bond in the theoretical amount.

Furthermore, a styrene-reduced molecular weight is obtained by the GPC analysis and a substantially theoretical degree of polymerization can be confirmed therefrom.

The present novel carbodiimide can be polymerized alone or in combination with other compounds having olefinic double bonds, namely polymerizable unsaturated monomers or oligomers or unsaturated resins, with a light, heat or a polymerization initiator if necessary, to produce a resin of higher strength. By the polymerization, there can also be obtained a novel polymer high in hydrolysis stability, heat resistance, water resistance, chemical resistance and adhesiveness to various substrates; in other words, conventional resins having said properties can be modified by the present carbodiimide.

Incidentally, among the above-mentioned compounds having olefinic double bonds, the polymerizable unsaturated monomers or oligomers include such monomers as ethylene, propylene, styrene, isoprene, butadiene, acrylonitrile, vinyl chloride, vinylidene chloride, methacrylic acid, methylmethacrylic acid, acrylic acid, vinyl acetate and the like and oligomers thereof, and the above unsaturated resins include unsaturated polyester resins.

When the carbodiimide of the present invention is polymerized with, for example, an unsaturated polyester resin, the strength and hydrolysis resistance of the unsaturated polyester resin can be enhanced. When the present carbodiimide is added to a resin having a group capable of reacting with carbodiimide group and they are subjected to polymerization, the water resistance and chemical resistance of the resin can be enhanced.

Moreover, when the above compound having, in the molecule, at least one polymerizable unsaturated bond and at least one group capable of reacting with isocyanate group has a hydrophilic group (for example, ethylene oxide, propylene oxide, amine, quaternary ammonium salt, sulfonic acid or the like) in the molecule, a hydrophilic unsaturated carbodiimide can be obtained by controlling the degree of polymerization of the carbodiimide so as to fall within an appropriate range, and this hydrophilic unsaturated carbodiimide is useful as a cross-linking agent for hydrophilic resins.

### DESCRIPTION OF THE PREFERRED EMBODIMENTS

Examples are shown below to explain the present invention in more detail.

### Synthesis of carbodiimide-modified diisocyanates

In a 5-liter reactor were placed 3.0 kg of 4,4'-dicyclohexylmethane diisocyanate (HMDI) and 15 g of a carbodiimidization catalyst (3-methyl-1-phenyl-2-phosphorene-1-oxide, the same applying to the synthesis examples appearing hereinafter), and they were subjected to reaction at 185°C for 10 hours while being stirred at 200 rpm by means of a mechanical stirrer, to obtain a carbodiimide having a degree of polymerization (n) of 3.2 and having terminal isocyanate groups (referred to hereinafter as Synthesis No. A).

The same procedure as above was repeated, except that the amounts of the diisocyanate and the catalyst and the reaction time were changed as shown in Table 1, to obtain carbodiimides (referred to hereinafter as Synthesis Nos. B to L).

**Table 1**

| Synthesis No. | Isocyanate | Amount (kg) | Catalyst (g) | Reaction time (hr) | Degree of polymerization (n) | Molecular weight | Solvent/Amount (g) (g) |
|---|---|---|---|---|---|---|---|
| A | HMDI | 3.0 | 15 | 10 | 3.2 | 960 | |
| B | HMDI | 3.0 | 15 | 15 | 10.5 | 2551 | |
| C | IPDI | 3.0 | 15 | 12 | 3.1 | 775 | |
| D | IPDI | 3.0 | 15 | 16 | 10.2 | 2041 | |
| E | TMXDI | 3.0 | 60 | 10 | 3.5 | 944 | |
| F | TMXDI | 3.0 | 60 | 15 | 12.1 | 2664 | |
| G | TIDI | 3.0 | 3 | 7 | 2.9 | 966 | |
| H | TIDI | 3.0 | 3 | 15 | 9.7 | 2657 | |
| I | MDI | 1.0 | 0.5 | 4 | 5.2 | 1323 | THF/900 Solid content 8.66 wt.% |
| J | MDI | 1.0 | 1 | 2 | 10.7 | 2457 | THF/900 Solid content 8.54 wt.% |
| K | TDI | 1.0 | 0.5 | 4 | 4.6 | 7730 | Perclene/900 Solid content 8.09 wt.% |
| L | TDI | 1.0 | 1 | 3 | 12.0 | 1736 | Perclene/900 Solid content 7.85 wt.% |

In Table 1, the abbreviations of diisocyanates have the following meanings, and the degree of polymerization and molecular weight were calculated from NCO % determined by a titration method:
- HMDI:: 4,4'-Dicyclohexylmethane diisocyanate
- TMXDI:: Tetramethylxylylene diisocyanate
- IPDI:: Isophorone diisocyanate
- MDI:: 4,4'-Diphenylmethane diisocyanate
- TDI:: Tolylene diisocyanate
- TIDI:: Triisopropylidenephenyl diisocyanate

### Example 1

To 96 g of the carbodiimide-modified diisocyanate of Synthesis No. A were added 50 g of toluene and 23.2 g of 2-hydroxyethyl acrylate (a double bond compound) corresponding to one equivalent of isocyanate, and they were subjected to reaction at 95°C for 5 hours while being stirred at 200 rpm using a mechanical stirrer, to synthesize a carbodiimide having double bonds and a molecular weight of 1,260 [the styrene-reduced molecular weight as measured by gel permeation chromatography (GPC) (the same applies to Examples appearing hereinafter)].

### Examples 2 to 27

In the same manner as in Example 1, except that the kind and amount of the double bond compound were changed as shown in Table 2, carbodiimides having double bonds and having the molecular weights shown in Table 2 were synthesized.

### Examples 28 to 54

In the same manner as in Examples 1 to 27, except that the solvent was not used, carbodiimides having double bonds and having the molecular weights shown in Table 2 were synthesized.

**Table 2**

| Example No. | Double-bond compd. | Amount (g) | Molecular weight | Example No. | Double-bond compd. | Amount (g) | Molecular weight |
|---|---|---|---|---|---|---|---|
| 1 | DBC-1 | 23.2 | 1260 | 28 | DBC-1 | 23.2 | 1260 |
| 2 | DBC-2 | 26.0 | 1290 | 29 | DBC-2 | 26.0 | 1290 |
| 3 | DBC-3 | 26.0 | 1310 | 30 | DBC-3 | 26.0 | 1310 |
| 4 | DBC-4 | 28.8 | 1320 | 31 | DBC-4 | 28.8 | 1320 |
| 5 | DBC-5 | 59.7 | 1600 | 32 | DBC-5 | 59.7 | 1600 |
| 6 | DBC-6 | 62.4 | 1700 | 33 | DBC-6 | 62.4 | 1700 |
| 7 | DBC-7 | 25.6 | 1260 | 34 | DBC-7 | 25.6 | 1260 |
| 8 | DBC-8 | 28.4 | 1270 | 35 | DBC-8 | 28.4 | 1270 |
| 9 | DBC-9 | 11.6 | 1160 | 36 | DBC-9 | 11.6 | 1160 |
| 10 | DBC-10 | 14.4 | 1180 | 37 | DBC-10 | 14.4 | 1180 |
| 11 | DBC-11 | 17.2 | 1190 | 38 | DBC-11 | 17.2 | 1190 |
| 12 | DBC-14 | 19.8 | 1200 | 39 | DBC-14 | 19.8 | 1200 |
| 13 | DBC-15 | 19.4 | 1190 | 40 | DBC-15 | 19.4 | 1190 |
| 14 | DBC-16 | 27.8 | 1270 | 41 | DBC-16 | 27.8 | 1270 |
| 15 | DBC-18 | 19.6 | 2200 | 42 | DBC-18 | 19.6 | 2200 |
| 16 | DBC-19 | 25.2 | 1250 | 43 | DBC-19 | 25.2 | 1250 |
| 17 | DBC-20 | 22.8 | 1220 | 44 | DBC-20 | 22.8 | 1220 |
| 18 | DBC-21 | 26.8 | 1250 | 45 | DBC-21 | 26.8 | 1250 |
| 19 | DBC-22 | 20.4 | 1180 | 46 | DBC-22 | 20.4 | 1180 |
| 20 | DBC-23 | 51.3 | 1490 | 47 | DBC-23 | 51.3 | 1490 |
| 21 | DBC-24 | 70.0 | 1970 | 48 | DBC-24 | 70.0 | 1970 |
| 22 | DBC-25 | 100.0 | 2190 | 49 | DBC-25 | 100.0 | 2190 |
| 23 | DBC-26 | 74.0 | 1720 | 50 | DBC-26 | 74.0 | 1720 |
| 24 | DBC-27 | 32.4 | 1700 | 51 | DBC-27 | 32.4 | 1700 |
| 25 | DBC-28 | 90.0 | 2290 | 52 | DBC-28 | 90.0 | 2290 |
| 26 | DBC-29 | 45.6 | 2030 | 53 | DBC-29 | 45.6 | 2030 |
| 27 | DBC-30 | 84.0 | 1990 | 54 | DBC-30 | 84.0 | 1990 |

In Table 2, the symbols of the double-bond compounds have the following meanings (the same applies to Examples appearing hereinafter):
DBC-1: 2-Hydroxyethyl acrylate
DBC-2: 2-Hydroxyethyl methacrylate
DBC-3: 2-Hydroxypropyl acrylate
DBC-4: 2-Hydroxypropyl methacrylate
DBC-5: Pentaerythritol triacrylate
DBC-6: Pentaerythritol trimethacrylate
DBC-7: Glycidyl acrylate
DBC-8: Glycidyl methacrylate
DBC-9: Allyl alcohol
DBC-10: 3-Buten-1-ol
DBC-11: 4-Penten-1-ol
DBC-12: Allylamine
DBC-13: N-methylallylamine
DBC-14: N-ethyl-2-methylallylamine
DBC-15: Diallylamine
DBC-16: Allylcyclohexylamine
DBC-17: Butadiene monoxide
DBC-18: 1,2-Epoxy-5-hexene
DBC-19: 1,2-Epoxy-7-octene
DBC-20: Allyl glycidyl ether
DBC-21: 2-Allylphenol
DBC-22: 2-Allyloxyethanol
DBC-23: Pentaerythritol triallyl ether
DBC-24: Polyethylene glycol monomethacrylate
DBC-25: Polypropylene glycol monomethacrylate
DBC-26: Polyethylene glycol monoacrylate
DBC-27: 2-Sulfoethyl methacrylate
DBC-28: Polyethylene glycol monoacrylate
DBC-29: 2-Hydroxy-1,3-dimethacryloxypropane
DBC-30: Polypropylene glycol monoacrylate

### Examples 55 to 81

In the same manner as in Examples 28 to 54, except that the carbodiimide-modified diisocyanate was replaced by 255.1 g of that of Synthesis No. B, carbodiimides having double bonds and having the molecular weights shown in Table 3 were synthesized.

### Examples 82 to 108

In The same manner as in Examples 28 to 54, except that the carbodiimide-modified diisocyanate was replaced by 77.5 g of that of Synthesis No. C, carbodiimides having double bonds and having the molecular weights shown in Table 3 were synthesized.

**Table 3**

| Example No. | Double-bond compd. | Amount (g) | Molecular weight | Example No. | Double-bond compd. | Amount (g) | Molecular weight |
|---|---|---|---|---|---|---|---|
| 55 | DBC-1 | 23.2 | 2960 | 82 | DBC-1 | 23.2 | 1060 |
| 56 | DBC-2 | 26.0 | 2980 | 83 | DBC-2 | 26.0 | 1090 |
| 57 | DBC-3 | 26.0 | 3010 | 84 | DBC-3 | 26.0 | 1120 |
| 58 | DBC-4 | 28.8 | 2990 | 85 | DBC-4 | 28.8 | 1130 |
| 59 | DBC-5 | 59.7 | 3260 | 86 | DBC-5 | 59.7 | 1410 |
| 60 | DBC-6 | 62.4 | 3380 | 87 | DBC-6 | 62.4 | 1500 |
| 61 | DBC-7 | 25.6 | 2910 | 88 | DBC-7 | 25.6 | 1060 |
| 62 | DBC-8 | 28.4 | 2890 | 89 | DBC-8 | 28.4 | 1080 |
| 63 | DBC-9 | 11.6 | 2900 | 90 | DBC-9 | 11.6 | 960 |
| 64 | DBC-10 | 14.4 | 2890 | 91 | DBC-10 | 14.4 | 980 |
| 65 | DBC-11 | 17.2 | 2890 | 92 | DBC-11 | 17.2 | 1000 |
| 66 | DBC-14 | 19.8 | 2860 | 93 | DBC-14 | 19.8 | 1010 |
| 67 | DBC-15 | 19.4 | 2830 | 94 | DBC-15 | 19.4 | 990 |
| 68 | DBC-16 | 27.8 | 2910 | 95 | DBC-16 | 27.8 | 1080 |
| 69 | DBC-18 | 19.6 | 3860 | 96 | DBC-18 | 19.6 | 2000 |
| 70 | DBC-19 | 25.2 | 2910 | 97 | DBC-19 | 25.2 | 1060 |
| 71 | DBC-20 | 22.8 | 2860 | 98 | DBC-20 | 22.8 | 1030 |
| 72 | DBC-21 | 26.8 | 2880 | 99 | DBC-21 | 26.8 | 1060 |
| 73 | DBC-22 | 20.4 | 2810 | 100 | DBC-22 | 20.4 | 990 |
| 74 | DBC-23 | 51.3 | 3100 | 101 | DBC-23 | 51.3 | 1300 |
| 75 | DBC-24 | 70.0 | 4070 | 102 | DBC-24 | 70.0 | 1720 |
| 76 | DBC-25 | 100.0 | 4160 | 103 | DBC-25 | 100.0 | 1960 |
| 77 | DBC-26 | 74.0 | 3350 | 104 | DBC-26 | 74.0 | 1530 |
| 78 | DBC-27 | 32.4 | 3970 | 105 | DBC-27 | 32.4 | 1430 |
| 79 | DBC-28 | 90.0 | 4610 | 106 | DBC-28 | 90.0 | 2030 |
| 80 | DBC-29 | 45.6 | 4640 | 107 | DBC-29 | 45.6 | 1730 |
| 81 | DBC-30 | 84.0 | 3900 | 108 | DBC-30 | 84.0 | 1770 |

### Examples 109 to 135

In the same manner as in Examples 28 to 54, except that the carbodiimide-modified diisocyanate was replaced by 204.1 g of that of Synthesis No. D, carbodiimides having double bonds and having the molecular weights shown in Table 4 were synthesized.

**Table 4**

| Example No. | Double-bond compound | Amount (g) | Molecular weight |
|---|---|---|---|
| 109 | DBC-1 | 23.2 | 2420 |
| 110 | DBC-2 | 26.0 | 2440 |
| 111 | DBC-3 | 26.0 | 2470 |
| 112 | DBC-4 | 28.8 | 2460 |
| 113 | DBC-5 | 59.7 | 2730 |
| 114 | DBC-6 | 62.4 | 2840 |
| 115 | DBC-7 | 25.6 | 2380 |
| 116 | DBC-8 | 28.4 | 2370 |
| 117 | DBC-9 | 11.6 | 2340 |
| 118 | DBC-10 | 14.4 | 2340 |
| 119 | DBC-11 | 17.2 | 2340 |
| 120 | DBC-14 | 19.8 | 2330 |
| 121 | DBC-15 | 19.4 | 2300 |
| 122 | DBC-16 | 27.8 | 2380 |
| 123 | DBC-18 | 19.6 | 3330 |
| 124 | DBC-19 | 25.2 | 2380 |
| 125 | DBC-20 | 22.8 | 2340 |
| 126 | DBC-21 | 26.8 | 2360 |
| 127 | DBC-22 | 20.4 | 2290 |
| 128 | DBC-23 | 51.3 | 2580 |
| 129 | DBC-24 | 70.0 | 3400 |
| 130 | DBC-25 | 100.0 | 3530 |
| 131 | DBC-26 | 74.0 | 2830 |
| 132 | DBC-27 | 32.4 | 3240 |
| 133 | DBC-28 | 90.0 | 3870 |
| 134 | DBC-29 | 45.6 | 3800 |
| 135 | DBC-30 | 84.0 | 3290 |

### Examples 136 to 165

In the same manner as in Examples 28 to 54, except that the carbodiimide-modified diisocyanate was replaced by 94.4 g of that of Synthesis No. E, the double bond compound was replaced by those shown in Table 5 in the amounts shown in Table 5 and the reaction was carried out at 50°C for 8 hours (in Examples 147 to 151, for 3 hours), carbodiimides having double bonds and having the molecular weights shown in Table 5 were synthesized.

### Example Nos. 166 to 195

In the same manner as in Example Nos. 136 to 165, except that the carbodiimide-modified diisocyanate was replaced by 266.4 g of that of Synthesis No. F (provided that when the double bond compounds DBC-12 - DBC-16 were used, the reaction time was 3 hours and in the other cases, the reaction time was 8 hours), carbodiimides having double bonds and having the molecular weights shown in Table 5 were synthesized.

**Table 5**

| Example No. | Double-bond compd. | Amount (g) | Molecular weight | Example No. | Double-bond compd. | Amount (g) | Molecular weight |
|---|---|---|---|---|---|---|---|
| 136 | DBC-1 | 23.2 | 1240 | 166 | DBC-1 | 23.2 | 3080 |
| 137 | DBC-2 | 26.0 | 1270 | 167 | DBC-2 | 26.0 | 3110 |
| 138 | DBC-3 | 26.0 | 1300 | 168 | DBC-3 | 26.0 | 3130 |
| 139 | DBC-4 | 28.8 | 1310 | 169 | DBC-4 | 28.8 | 3110 |
| 140 | DBC-5 | 59.7 | 1580 | 170 | DBC-5 | 59.7 | 3380 |
| 141 | DBC-6 | 62.4 | 1680 | 171 | DBC-6 | 62.4 | 3500 |
| 142 | DBC-7 | 25.6 | 1240 | 172 | DBC-7 | 25.6 | 3030 |
| 143 | DBC-8 | 28.4 | 1250 | 173 | DBC-8 | 28.4 | 3010 |
| 144 | DBC-9 | 11.6 | 1150 | 174 | DBC-9 | 11.6 | 3020 |
| 145 | DBC-10 | 14.4 | 1160 | 175 | DBC-10 | 14.4 | 3010 |
| 146 | DBC-11 | 17.2 | 1180 | 176 | DBC-11 | 17.2 | 3010 |
| 147 | DBC-12 | 11.4 | 1160 | 177 | DBC-12 | 11.4 | 3050 |
| 148 | DBC-13 | 14.2 | 1170 | 178 | DBC-13 | 14.2 | 3030 |
| 149 | DBC-14 | 19.8 | 1180 | 179 | DBC-14 | 19.8 | 2980 |
| 150 | DBC-15 | 19.4 | 1170 | 180 | DBC-15 | 19.4 | 2940 |
| 151 | DBC-16 | 27.8 | 1250 | 181 | DBC-16 | 27.8 | 3030 |
| 152 | DBC-17 | 14.0 | 1170 | 182 | DBC-17 | 14.0 | 3040 |
| 153 | DBC-18 | 19.6 | 2180 | 183 | DBC-18 | 19.6 | 3970 |
| 154 | DBC-19 | 25.2 | 1240 | 184 | DBC-19 | 25.2 | 3030 |
| 155 | DBC-20 | 22.8 | 1200 | 185 | DBC-20 | 22.8 | 2980 |
| 156 | DBC-21 | 26.8 | 1230 | 186 | DBC-21 | 26.8 | 2990 |
| 157 | DBC-22 | 20.4 | 1170 | 187 | DBC-22 | 20.4 | 2920 |
| 158 | DBC-23 | 51.3 | 1470 | 188 | DBC-23 | 51.3 | 3210 |
| 159 | DBC-24 | 70.0 | 1950 | 189 | DBC-24 | 70.0 | 4220 |
| 160 | DBC-25 | 100.0 | 2170 | 190 | DBC-25 | 100.0 | 4300 |
| 161 | DBC-26 | 74.0 | 1710 | 191 | DBC-26 | 74.0 | 3470 |
| 162 | DBC-27 | 32.4 | 1670 | 192 | DBC-27 | 32.4 | 4130 |
| 163 | DBC-28 | 90.0 | 2270 | 193 | DBC-28 | 90.0 | 4770 |
| 164 | DBC-29 | 45.6 | 2000 | 194 | DBC-29 | 45.6 | 4830 |
| 165 | DBC-30 | 84.0 | 1970 | 195 | DBC-30 | 84.0 | 4040 |

### Examples 196 to 225

In the same manner as in Examples 136 to 165, except that the carbodiimide-modified diisocyanate was replaced by 96.6 g of that of Synthesis No. G (provided that when the double bond compounds DBC-12 - DBC-16 were used, the reaction time was 3 hours and in the other cases, the reaction time was 8 hours), carbodiimides having double bonds and having the molecular weights shown in Table 6 were synthesized.

### Examples 226 to 255

In the same manner as in Examples 136 to 165, except that the carbodiimide-modified diisocyanate was replaced by 265.7 g of that of Synthesis No. H (provided that when the double bond compounds DBC-12 - DBC-16 were used, the reaction time was 3 hours and in the other cases, the reaction time was 8 hours), carbodiimides having double bonds and having the molecular weights shown in Table 6 were synthesized.

**Table 6**

| Example No. | Double-bond compd. | Amount (g) | Molecular weight | Example No. | Double-bond compd. | Amount (g) | Molecular weight |
|---|---|---|---|---|---|---|---|
| 196 | DBC-1 | 23.2 | 1270 | 226 | DBC-1 | 23.2 | 3080 |
| 197 | DBC-2 | 26.0 | 1290 | 227 | DBC-2 | 26.0 | 3100 |
| 198 | DBC-3 | 26.0 | 1320 | 228 | DBC-3 | 26.0 | 3120 |
| 199 | DBC-4 | 28.8 | 1330 | 229 | DBC-4 | 28.8 | 3110 |
| 200 | DBC-5 | 59.7 | 1610 | 230 | DBC-5 | 59.7 | 3370 |
| 201 | DBC-6 | 62.4 | 1700 | 231 | DBC-6 | 62.4 | 3500 |
| 202 | DBC-7 | 25.6 | 1260 | 232 | DBC-7 | 25.6 | 3020 |
| 203 | DBC-8 | 28.4 | 1270 | 233 | DBC-8 | 28.4 | 3000 |
| 204 | DBC-9 | 11.6 | 1170 | 234 | DBC-9 | 11.6 | 3010 |
| 205 | DBC-10 | 14.4 | 1180 | 235 | DBC-10 | 14.4 | 3000 |
| 206 | DBC-11 | 17.2 | 1200 | 236 | DBC-11 | 17.2 | 3000 |
| 207 | DBC-12 | 11.4 | 1180 | 237 | DBC-12 | 11.4 | 3040 |
| 208 | DBC-13 | 14.2 | 1190 | 238 | DBC-13 | 14.2 | 3020 |
| 209 | DBC-14 | 19.8 | 1210 | 239 | DBC-14 | 19.8 | 2970 |
| 210 | DBC-15 | 19.4 | 1190 | 240 | DBC-15 | 19.4 | 2940 |
| 211 | DBC-16 | 27.8 | 1280 | 241 | DBC-16 | 27.8 | 3020 |
| 212 | DBC-17 | 14.0 | 1190 | 242 | DBC-17 | 14.0 | 3030 |
| 213 | DBC-18 | 19.6 | 2200 | 243 | DBC-18 | 19.6 | 3970 |
| 214 | DBC-19 | 25.2 | 1260 | 244 | DBC-19 | 25.2 | 3020 |
| 215 | DBC-20 | 22.8 | 1230 | 245 | DBC-20 | 22.8 | 2970 |
| 216 | DBC-21 | 26.8 | 1260 | 246 | DBC-21 | 26.8 | 2990 |
| 217 | DBC-22 | 20.4 | 1190 | 247 | DBC-22 | 20.4 | 2910 |
| 218 | DBC-23 | 51.3 | 1490 | 248 | DBC-23 | 51.3 | 3200 |
| 219 | DBC-24 | 70.0 | 1980 | 249 | DBC-24 | 70.0 | 4210 |
| 220 | DBC-25 | 100.0 | 2200 | 250 | DBC-25 | 100.0 | 4290 |
| 221 | DBC-26 | 74.0 | 1730 | 251 | DBC-26 | 74.0 | 3460 |
| 222 | DBC-27 | 32.4 | 1710 | 252 | DBC-27 | 32.4 | 4120 |
| 223 | DBC-28 | 90.0 | 2300 | 253 | DBC-28 | 90.0 | 4760 |
| 224 | DBC-29 | 45.6 | 2040 | 254 | DBC-29 | 45.6 | 4810 |
| 225 | DBC-30 | 84.0 | 2000 | 255 | DBC-30 | 84.0 | 4030 |

### Examples 256 to 285

In the same manner as in Examples 1 to 27, except that the carbodiimide-modified diisocyanate was replaced by 763.7 g of a solution of the carbodiimide-modified diisocyanate of Synthesis No. I in tetrahydrofuran (THF) and the double bond compound was replaced by one of the double bond compounds shown in Table 7 in the amount shown in Table 7, carbodiimides having double bonds and having the molecular weights shown in Table 7 were synthesized.

### Examples 286 to 315

In the same manner as in Examples 256 to 285, except that the carbodiimide-modified diisocyanate was replaced by 1,438.5 g of a solution of the carbodiimide-modified diisocyanate of Synthesis No. J in tetrahydrofuran (THF), carbodiimides having double bonds and having the molecular weights shown in Table 7 were synthesized.

**Table 7**

| Example No. | Double-bond compd. | Amount (g) | Molecular weight | Example No. | Double-bond compd. | Amount (g) | Molecular weight |
|---|---|---|---|---|---|---|---|
| 256 | DBC-1 | 11.6 | 1650 | 286 | DBC-1 | 11.6 | 2860 |
| 257 | DBC-2 | 13.0 | 1670 | 287 | DBC-2 | 13.0 | 2880 |
| 258 | DBC-3 | 13.0 | 1700 | 288 | DBC-3 | 13.0 | 2910 |
| 259 | DBC-4 | 14.4 | 1710 | 289 | DBC-4 | 14.4 | 2900 |
| 260 | DBC-5 | 29.8 | 1980 | 290 | DBC-5 | 29.8 | 3160 |
| 261 | DBC-6 | 31.2 | 2080 | 291 | DBC-6 | 31.2 | 3290 |
| 262 | DBC-7 | 12.8 | 1630 | 292 | DBC-7 | 12.8 | 2820 |
| 263 | DBC-8 | 14.2 | 1640 | 293 | DBC-8 | 14.2 | 2800 |
| 264 | DBC-9 | 5.8 | 1560 | 294 | DBC-9 | 5.8 | 2790 |
| 265 | DBC-10 | 7.2 | 1570 | 295 | DBC-10 | 7.2 | 2790 |
| 266 | DBC-11 | 8.6 | 1580 | 296 | DBC-11 | 8.6 | 2790 |
| 267 | DBC-12 | 5.7 | 1570 | 297 | DBC-12 | 5.7 | 2820 |
| 268 | DBC-13 | 7.1 | 1580 | 298 | DBC-13 | 7.1 | 2810 |
| 269 | DBC-14 | 9.9 | 1580 | 299 | DBC-14 | 9.9 | 2760 |
| 270 | DBC-15 | 9.7 | 1560 | 300 | DBC-15 | 9.7 | 2730 |
| 271 | DBC-16 | 13.9 | 1640 | 301 | DBC-16 | 13.9 | 2810 |
| 272 | DBC-17 | 7.0 | 1580 | 302 | DBC-17 | 7.0 | 2820 |
| 273 | DBC-18 | 9.8 | 2580 | 303 | DBC-18 | 9.8 | 3760 |
| 274 | DBC-19 | 12.6 | 1630 | 304 | DBC-19 | 12.6 | 2820 |
| 275 | DBC-20 | 11.4 | 1600 | 305 | DBC-20 | 11.4 | 2770 |
| 276 | DBC-21 | 13.4 | 1620 | 306 | DBC-21 | 13.4 | 2780 |
| 277 | DBC-22 | 10.2 | 1550 | 307 | DBC-22 | 10.2 | 2710 |
| 278 | DBC-23 | 25.6 | 1850 | 308 | DBC-23 | 25.6 | 3000 |
| 279 | DBC-24 | 35.0 | 2450 | 309 | DBC-24 | 35.0 | 3950 |
| 280 | DBC-25 | 50.0 | 2640 | 310 | DBC-25 | 50.0 | 4050 |
| 281 | DBC-26 | 37.0 | 2090 | 311 | DBC-26 | 37.0 | 3250 |
| 282 | DBC-27 | 16.2 | 2220 | 312 | DBC-27 | 16.2 | 3840 |
| 283 | DBC-28 | 45.0 | 2820 | 313 | DBC-28 | 45.0 | 4470 |
| 284 | DBC-29 | 22.8 | 2630 | 314 | DBC-29 | 22.8 | 4490 |
| 285 | DBC-30 | 42.0 | 2430 | 315 | DBC-30 | 42.0 | 3790 |

### Examples 316 to 345

In the same manner as in Examples 256 to 285, except that the carbodiimide-modified diisocyanate was replaced by 477.8 g of a solution of the carbodiimide-modified diisocyanate of Synthesis No. K in Perclene, carbodiimides having double bonds and having the molecular weights shown in Table 8 were synthesized.

### Examples 346 to 375

In the same manner as in Examples 256 to 285, except that the carbodiimide-modified diisocyanate was replaced by 1,105.7 g of a solution of the carbodiimide-modified diisocyanate of Synthesis No. I in Perclene, carbodiimides having double bonds and the molecular weights shown in Table 8 were synthesized.

**Table 8**

| Example No. | Double-bond compd. | Amount (g) | Molecular weight | Example No. | Double-bond compd. | Amount (g) | Molecular weight |
|---|---|---|---|---|---|---|---|
| 316 | DBC-1 | 11.6 | 8500 | 346 | DBC-1 | 11.6 | 2090 |
| 317 | DBC-2 | 13.0 | 8520 | 347 | DBC-2 | 13.0 | 2110 |
| 318 | DBC-3 | 13.0 | 8540 | 348 | DBC-3 | 13.0 | 2140 |
| 319 | DBC-4 | 14.4 | 8430 | 349 | DBC-4 | 14.4 | 2140 |
| 320 | DBC-5 | 29.8 | 8670 | 350 | DBC-5 | 29.8 | 2410 |
| 321 | DBC-6 | 31.2 | 8870 | 351 | DBC-6 | 31.2 | 2520 |
| 322 | DBC-7 | 12.8 | 8310 | 352 | DBC-7 | 12.8 | 2070 |
| 323 | DBC-8 | 14.2 | 8190 | 353 | DBC-8 | 14.2 | 2060 |
| 324 | DBC-9 | 5.8 | 8540 | 354 | DBC-9 | 5.8 | 2010 |
| 325 | DBC-10 | 7.2 | 8460 | 355 | DBC-10 | 7.2 | 2010 |
| 326 | DBC-11 | 8.6 | 8400 | 356 | DBC-11 | 8.6 | 2020 |
| 327 | DBC-12 | 5.7 | 8640 | 357 | DBC-12 | 5.7 | 2030 |
| 328 | DBC-13 | 7.1 | 8520 | 358 | DBC-13 | 7.1 | 2020 |
| 329 | DBC-14 | 9.9 | 8260 | 359 | DBC-14 | 9.9 | 2010 |
| 330 | DBC-15 | 9.7 | 8170 | 360 | DBC-15 | 9.7 | 1990 |
| 331 | DBC-16 | 13.9 | 8260 | 361 | DBC-16 | 13.9 | 2070 |
| 332 | DBC-17 | 7.0 | 8560 | 362 | DBC-17 | 7.0 | 2030 |
| 333 | DBC-18 | 9.8 | 9260 | 363 | DBC-18 | 9.8 | 3010 |
| 334 | DBC-19 | 12.6 | 8310 | 364 | DBC-19 | 12.6 | 2060 |
| 335 | DBC-20 | 11.4 | 8210 | 365 | DBC-20 | 11.4 | 2020 |
| 336 | DBC-21 | 13.4 | 8180 | 366 | DBC-21 | 13.4 | 2040 |
| 337 | DBC-22 | 10.2 | 8090 | 367 | DBC-22 | 10.2 | 1970 |
| 338 | DBC-23 | 25.6 | 8340 | 368 | DBC-23 | 25.6 | 2270 |
| 339 | DBC-24 | 35.0 | 10920 | 369 | DBC-24 | 35.0 | 2990 |
| 340 | DBC-25 | 50.0 | 10590 | 370 | DBC-25 | 50.0 | 3150 |
| 341 | DBC-26 | 37.0 | 8650 | 371 | DBC-26 | 37.0 | 2520 |
| 342 | DBC-27 | 16.2 | 11380 | 372 | DBC-27 | 16.2 | 2810 |
| 343 | DBC-28 | 45.0 | 12130 | 373 | DBC-28 | 45.0 | 3420 |
| 344 | DBC-29 | 22.8 | 13130 | 374 | DBC-29 | 22.8 | 3300 |
| 345 | DBC-30 | 42.0 | 10120 | 375 | DBC-30 | 42.0 | 2920 |

Incidentally, the properties of the carbodiimides having double bonds of the present invention synthesized as mentioned above were as follows.

### Examples 1 to 27

Pale yellow liquid (solution)
   Examples 28 to 54 (excluding Examples 48, 49, 50, 52 and 54) and Examples 55 to 81 (excluding Examples 75, 76, 77, 79 and 81)
Pale yellow solid having softening point of 35°C to 45°C Examples 82 to 108 (excluding Examples 102, 103, 104, 106 and 108) and Examples 109 to 135 (excluding Examples 129, 130, 131, 133 and 135)
Page yellow solid having softening point of 35°C to 55°C Examples 136 to 195
Brown liquid having high viscosity
   Examples 196 to 225 (excluding Examples 219, 220, 221, 223, 225, 249, 250, 251, 253 and 255)
Pale yellow solid having softening point of 45°C to 65°C Examples 256 to 375
Pale yellow liquid (solution)
   Examples 48, 49, 50, 52, 54, 75, 76, 77, 79, 81, 102, 103, 104, 106, 108, 129, 130, 131, 133, 135, 219, 220, 221, 223, 225, 249, 250, 251, 253 and 255
Pale yellow liquid having high viscosity

Moreover, the structures of the carbodiimides having double bonds of the present invention were confirmed as follows.

### 1. IR

The disappearance of the peak of isocyanate at 2200-2300 cm⁻¹ was observed, whereby it was confirmed that the isocyanate had reacted quantitatively with an amount equivalent thereto of a group capable of reacting with the isocyanate group. Moreover, the peak of carbodiimide at 2100-2200 cm⁻¹ was observed, and from the fact that carbodiimide group was present in the resulting compound and the ratio of the peak of carbodiimide to the C-H peak due to alkyl in the vicinity of 3000-3500 cm⁻¹ had not changed, it was confirmed that no reduction of the amount of carbodiimide was seen and the theoretical amount of carbodiimide was present.

Furthermore, in the Examples in which the double bond compounds DBC-7, 8, 17, 18, 19 and 20 were used, peaks resulting from reaction between epoxy and isocyanate were confirmed at 1700-1800 cm⁻¹, in Examples in which the double bond compounds DBC-1, 2, 3, 4, 5, 6, 9, 10, 11, 21, 22, 23, 24, 25, 26, 27, 28, 29 and 30 were used, peaks resulting from reaction between alcohol and isocyanate were confirmed at 1700-1800 cm⁻¹, and in the Examples in which the double bond compounds DBC-12, 13, 14, 15 and 16 were used, peaks resulting from reaction between amine and isocyanate were confirmed at 1400-1700 cm⁻¹.

### 2. NMR

The proton of H₂C=CH- or H₂C=C(R)- was observed at 4.5-7 ppm. Further, the amount of proton bonded to the carbon of the double bond was calculated from the integration value of proton peak of methyl and/or methylene group in the molecule, whereby the presence of a theoretical amount of unsaturated bond was confirmed.

### Assay of hydrolysis stabilizing effect of the present carbodiimide having double bonds

(1) A composition composed of the following components was injected in an amount of 20 g into a circle-shaped mold having a diameter of 50 mm and cured at 100°C for 6 hours.

| | |
|---|---|
| Unsaturated polyester resin (Polymal 6316, registered trade mark of Takeda Chemical Industries, Ltd.) | 100 g |
| Carbodiimide having double bonds synthesized in one of Examples 1 to 375 | 5 g |
| Benzoyl peroxide (BPO) | 1 g |

(2) In the same manner as above, a composition composed of the following components was injected in an amount of 20 g into a circle-shaped mold having a diameter of 50 mm and allowed to gel at 30°C for 2 hours and then cured at 50°C for 24 hours.

| | |
|---|---|
| Unsaturated polyester resin (Polyset 5595 PT-L, registered trade mark of Hitachi Chemical Co., Ltd.) | 100 g |
| Carbodiimide having double bonds synthesized in one of Examples 1 to 375 | 5 g |
| Methyl ethyl ketone peroxide | 1 g |

(3) In the same manner as in (1) and (2) above, except that the carbodiimide having double bonds synthesized in Examples 1 to 375 was not added, respective blanks were prepared.

Each of the resin molded articles obtained under the above conditions was immersed in hot water of 90°C, and the state of the resin after 240 hours was visually observed to evaluate blister and cracking, thereby finding that the resin molded articles each containing the carbodiimide having double bonds synthesized in one of Examples 1 to 375 had a smooth surface and the blanks free from the carbodiimide had cracks on the surface and in the interior.

### Assay of effect of resin cross-linking of the present carbodiimide havinq double bonds

(1) A blue pigment was added to an aqueous acrylic resin (Joncryl 61J, registered trade mark of JOHNSON & JOHNSON; acid value: 195; aqueous solution of a solid content of 30.5%), and thereto was further added one of the carbodiimides having double bonds synthesized in Examples 48, 49, 50, 52, 54, 75, 76, 77, 79, 81, 102, 103, 104, 106, 108, 129, 130, 131, 133, 135, 159, 160, 161, 163, 165, 189, 190, 191, 193, 195, 219, 220, 221, 223, 225, 249, 250, 251, 253, 255, 279, 280, 281, 283, 285, 309, 310, 311, 313, 315, 339, 340, 341, 343, 345, 369, 370, 371, 373 and 375 in an equivalent (the carboxylic acid of the resin and the carbodiimide group were in equivalent relation), and thereafter, the resulting mixture was formed into a PET film having a thickness of 100 µm by a bar coater and then the film was dried at 80 °C for 30 minutes to prepare a film having a thickness of 30 µm.
(2) A blue pigment was added to an oily acrylic resin (Joncryl J586, registered trade mark of JOHNSON & JOHNSON; acid value: 105; a toluene solution having a solid content of 20%), and thereto was further added one of the carbodiimides having double bonds synthesized in Examples 1 to 375 in an equivalent (the carboxylic acid of the resin and the carbodiimide group were in equivalent relation), after which the resulting mixture was formed into a PET film having a thickness of 100 µm by a bar coater. The film was dried at 100°C for 10 minutes to prepare a film having a thickness of 20 µm.
(3) In the same manner as in (1) and (2) above, except that the carbodiimide having double bonds was not added, respective blanks were prepared.

Each of the films obtained as mentioned above was lightly rubbed with absorbent wadding impregnated with 28% ammonia (solvent A) or a water-methanol (4/6) mixture (solvent B) to examine the number of rubbing strokes until crocking was caused, to find that each film containing the present carbodiimide having double bonds did not cause crocking even when rubbed more than 100 strokes with each of the absorbent waddings impregnated with solvents, in both cases of the aqueous and oily resins. However, in the case of the oily resin film free from the present carbodiimide having double bonds, the number of rubbing strokes for crocking was 20 in the case of solvent A and 5 in the case of solvent B. Similarly, in the aqueous resin film free from the present carbodiimide having double bonds, the number of rubbing strokes for crocking was 3 in the case of solvent A and 1 in the case of solvent B.

The present carbodiimide having double bonds is represented by the formula (1):

R₁-X-(R₂-NCN)ₙ-R₂-X-R₃ (1)

and this carbodiimide exhibits excellent performance as a hydrolysis stabilizer for ester bond-containing resins or as a cross-linking agent for resins having a group capable of reacting with carbodiimide group or for compounds having an olefinic double bond(s).

## Claims

1. A carbodiimide having double bonds, which is represented by the formula (1):
R₁-X-(R₂-NCN)ₙ-R₂-X-R₃ (1)
wherein R₁ and R₃ may be the same as or different from each other and each represents the organic residue having 3 to 40 carbon atoms, of a compound having at least one olefinic double bond and a functional group capable of reacting with an isocyanate group, formed by removing the functional group therefrom; R₂ represents the organic residue of a diisocyanate formed by removing the isocyanate group therefrom; plural R₂ groups may be different from one another; X represents a functional group formed by reaction between the above isocyanate group and the above functional group capable of reacting with isocyanate group; and n is a numerical value between 1.0 and 20.0.

2. The carbodiimide having double bonds according to claim 1, wherein R₂ is the organic residue resulting from 4,4' - dicyclohexylmethane diisocyanate, tetramethylxylylene diisocyanate or isophorone diisocyanate.

3. The carbodiimide having double bonds according to claim 1, wherein R₂ is the organic residue resulting from 4,4' - diphenylmethane diisocyanate, tolylene diisocyanate or triisopropylidenephenyl diisocyanate.

4. The carbodiimide having double bonds according to claim 1, wherein at least one of R₁ and R₃ is the organic residue resulting from 2-hydroxyethyl acrylate, 2-hydroxyethyl methacrylate, 2-hydroxypropyl acrylate, 2-hydroxypropyl methacrylate, pentaerythritol triacrylate, pentaerythritol trimethacrylate, glycidyl acrylate, glycidyl methacrylate, allyl alcohol, 3-buten-1-ol, 4-penten-1-ol, allylamine, N-methylallylamine, N-ethyl-2-methylallylamine, diallylamine, allylcyclohexylamine, butadiene monoxide, 1,2-epoxy-5-hexene, 1,2-epoxy-7-octene, allyl glycidyl ether, 2-allylphenol, 2-allyloxyethanol, pentaerythritol triallyl ether, polyethylene glycol monomethacrylate, polypropylene glycol monomethacrylate, polyethylene glycol monoacrylate, 2-sulfoethyl methacrylate, 2-hydroxy-1,3-dimethacryloxypropane or polypropylene glycol monoacrylate.

5. A hydrolysis stabilizer for an ester bond-containing resin, which comprises as a main component a carbodiimide according to one of the claims 1 - 4.

6. The hydrolysis stabilizer according to claim 5, wherein the ester bond-containing resin is a polyester resin or a polyesterpolyurethane resin.

7. A cross-linking agent for a resin having a group capable of reacting with carbodiimide group, which comprises as a main component a carbodiimide according to one of the claims 1 - 4.

8. A cross-linking agent for a compound having an olefinic double bond(s), which comprises as a main component a carbodiimide according to one of the claims 1 - 4.

## Patentansprüche

1. Carbodiimid mit Doppelbindungen, welches durch die Formel (1) wiedergegeben wird:
R₁-X-(R₂-NCN)ₙ-R₂-X-R₃ (1)
**dadurch gekennzeichnet, dass** R₁ und R₃ gleich sein oder sich voneinander unterscheiden sein können und jedes den organischen Rest mit 3 bis 40 Kohlenstoffatomen einer Verbindung mit mindestens einer olefinischen Doppelbindung und einer funktionellen Gruppe, welche mit einer durch Entfernen der funktionellen Gruppe daraus gebildeten Isocyanat-Gruppe reagieren kann, darstellt; R₂ den organischen Rest eines durch Entfernen der Isocyanat-Gruppe daraus gebildeten Diisocyanats darstellt; mehrere R₂-Gruppen sich voneinander unterscheiden können; X eine durch Reaktion zwischen der obigen Isocyanat-Gruppe und der obigen funktionellen Gruppe, welche mit der Isocyanat-Gruppe reagieren kann, gebildete funktionelle Gruppe darstellt; und n ein numerischer Wert zwischen 1,0 und 20,0 ist.

2. Carbodiimid mit Doppelbindungen nach Anspruch 1, **dadurch gekennzeichnet, dass** R₂ der organische Rest ist, welcher sich aus 4,4'-Dicyclohexylmethan-Diisocyanat, Tetramethylxylylen-Diisocyanat oder Isophoron-Diisocyanat ergibt.

3. Carbodiimid mit Doppelbindungen nach Anspruch 1, **dadurch gekennzeichnet, dass** R₂ der organische Rest ist, welcher sich aus 4,4'-Diphenylmethan-Diisocyanat, Tolylen-Diisocyanat oder Triisopropylidenphenyl-Diisocyanat ergibt.

4. Carbodiimid mit Doppelbindungen nach Anspruch 1, **dadurch gekennzeichnet, dass** mindestens eines von R₁ und R₃ der organische Rest ist, der sich aus 2-Hydroxyethyl-Acrylat, 2-Hydroxyethyl-Methacrylat, 2-Hydroxypropyl-Acrylat, 2-Hydroxypropyl-Methacrylat, Pentaerythritol-Triacrylat, Pentaerytritol-Trimethacrylat, Glycidyl-Acrylat, Glycidyl-Methacrylat, Allyl-Alkohol, 3-Buten-1-ol, 4-Penten-1-ol, Allylamin, N-Methylallylamin, N-Ethyl-2-Methylallylamin, Diallylamin, Allylcyclohexylamin, Butadien-Monoxid, 1,2-Epoxy-5-Hexen, 1,2-Epoxy-7-Octen, Allyl-Glycidylether, 2-Allylphenol, 2-Allyloxyethanol, Pentaerythritoi-Triallylether, Polyethylen-Glykol-Monomethacrylat, Polypropylen-Glykol-Monomethacrylat, Polyethylen-Glykol-Monoacrylat, 2-Sulfoethyl-Methacrylat, 2-Hydroxy-1,3-Dimethacryloxypropan oder Polypropylen-Glykol-Monoacrylat ergibt.

5. Hydrolyse-Stabilisator für ein esterbindungshaltiges Harz, welches als Hauptbestandteil ein Carbodiimid nach einem der Ansprüche 1 - 4 enthält.

6. Hydrolyse-Stabilisator nach Anspruch 5, **dadurch gekennzeichnet, dass** das esterbindungshaltige Harz ein Polyesterharz oder ein Polyesterpolyurethanharz ist.

7. Quervernetzendes Agens für ein Harz mit einer mit der Carbodiimid-Gruppe reaktionsfähigen Gruppe, welches als Hauptbestandteil ein Carbodiimid nach einem der Ansprüche 1 - 4 umfasst.

8. Quervernetzendes Agens für eine Verbindung mit einer olefinischen Doppelbindung/mit olefinischen Doppelbindungen, welches als Hauptbestandteil ein Carbodiimid nach einem der Ansprüche 1 - 4 umfasst.

## Revendications

1. Carbodiimide ayant des double liaisons, qui est représenté par la formule (1) :
R₁-X-(R₂-NCN)ₙ-R₂-X-R₃ (1)
où R₁ et R₃ peuvent être identiques ou différents l'un de l'autre et chacun représente le résidu organique ayant 3 à 40 atomes de carbone, d'un composé ayant au moins une double liaison oléfinique et un groupe fonctionnel capable de réagir avec un groupe isocyanate, formé en en enlevant le groupe fonctionnel ; R₂ représente le résidu organique d'un diisocyanate formé en en enlevant le groupe isocyanate ; plusieurs groupes R₂ peuvent être différents les uns des autres ; X représente un groupe fonctionnel formé par réaction entre le groupe isocyanate ci-dessus et le groupe fonctionnel ci-dessus capable de réagir avec le groupe isocyanate ; et n est une valeur numérique entre 1,0 et 20,0.

2. Carbodiimide ayant des double liaisons selon la revendication 1, où R₂ est le résidu organique résultant du 4,4'-dicyclohexylméthane diisocyanate, du tétraméthylxylylêne diisocyanate ou du diisocyanate d'isophorone.

3. Carbodiimide ayant des double liaisons selon la revendication 1, où R₂ est le résidu organique résultant du 4,4'-diphénylméthane diisocyanate, du diisocyanate de tolylène ou du diisocyanate de triisopropylidènephényle.

4. Carbodiimide ayant des double liaisons selon la revendication 1, où au moins l'un de R₂ et R₃ est le résidu organique résultant du 2-hydroxyéthyl acrylate, 2-hydroxyéthyl méthacrylate, 2-hydroxypropyl acrylate, 2-hydroxypropyl méthacrylate, triacrylate de pentaérythritol, triméthaacrylate de pentaérythritol, acrylate de glycidyle, méthacrylate de glycidyle, alcool allylique, 3-butèn-1-ol, 4-pentèn-1-ol, allylamine, N-méthylallylamine, N-éthyl-2-méthylallylamine, diallylamine, allylcyclohexylamine, monoxyde de butadiène, 1,2-époxy-5-hexène, 1,2-époxy-7-octène, allyl glycidyl éther, 2-allylphénol, 2-allyloxyéthanol, pentaérythritol triallyl éther, monométhacrylate de polyéthylène glycol, monométhacrylate de polypropylène glycol, monoacrylate de polyéthylène glycol, méthacrylate de 2-sulfoéthyle, 2-hydroxy-1, 3 diméthacryloxypropane ou monoacrylate de polypropylène glycol.

5. Stabilisant d'hydrolyse pour une résine contenant une liaison ester qui comprend comme composant principal un carbodiimide selon l'une des revendications 1-4.

6. Stabilisant d'hydrolyse selon la revendication 5, où la résine contenant une liaison ester est une résine polyester ou une résine polyesterpolyuréthanne.

7. Agent réticulant pour une résine ayant un groupe capable de réagir avec un groupe carbodiimide, qui comprend comme composant principal un carbodiimide selon l'une quelconque des revendications 1-4.

8. Agent réticulant pour un composé ayant une ou des double liaisons oléfiniques qui comprend comme composant principal un carbodiimide selon l'une quelconque des revendications 1-4.
